# EUROPEAN PATENT SPECIFICATION

(11) **EP 2 701 728 B1**
(45) Date of publication and mention of the grant of the patent: **06.05.2015**
(21) Application number: 13709511.3
(22) Date of filing: 05.03.2013
(51) Int. Cl.: A61K 38/22, A61K 38/33, A61K 38/57, A61K 47/42, A61P 31/14

(54) **FORMULATION COMPRISING CRH AND ALPHA-2-MACROGLOBULIN**
FORMULIERUNG MIT CRH- UND ALPHA-2-MAKROGLOBULIN
FORMULATION COMPRENANT CRH ET L'ALPHA-2-MACROGLOBULINE

(30) Priority: 25.06.2012 GB 201211247; 03.12.2012 GB 201221741
(43) Date of publication of application: 05.03.2014
(73) Proprietor: Aimsco Limited, East Sussex BN21 4RL (GB)
(72) Inventor: MCINTOSH, Deirdre.P., Eastbourne East Sussex BN21 4RL (GB); SHOTTON, David.J., Eastbourne East Sussex BN21 4RL (GB); HAQ, Syed. Ebadat., Eastbourne East Sussex BN21 4RL (GB)
(74) Representative: Zvesper, Thomas
(86) International application number: PCT/GB2013/050539
(87) International publication number: WO 2014/001749

(56) References cited:
- WO-A2-2006/021814
- R. B. ANDERSON ET AL: "2-Macroglobulin binds CpG oligodeoxynucleotides and enhances their immunostimulatory properties by a receptor-dependent mechanism", JOURNAL OF LEUKOCYTE BIOLOGY, vol. 83, no. 2, 1 January 2007 (2007-01-01), pages 381-392, XP055061155, ISSN: 0741-5400, DOI: 10.1189/jlb.0407236
- ALEXANDER GOURINE ET AL: "Role of alpha(2)-macroglobulin in fever and cytokine responses induced by lipopolysaccharide in mice.", AMERICAN JOURNAL OF PHYSIOLOGY. REGULATORY, INTEGRATIVE AND COMPARATIVE PHYSIOLOGY, vol. 283, no. 1, 1 July 2002 (2002-07-01), pages R218-R226, XP055061491, ISSN: 0363-6119, DOI: 10.1152/ajpregu.00746.2001
- SAPHIER P W ET AL: "A comparison of the clearance of ovine and human corticotrophin-releasing hormone (CRH) in man and sheep: a possible role for CRH-binding protein", JOURNAL OF ENDOCRINOLOGY, SOCIETY FOR ENDOCRINOLOGY, GB, vol. 133, no. 3, 1 June 1992 (1992-06-01), pages 487-495, XP009169119, ISSN: 0022-0795, DOI: 10.1677/JOE.0.1330487

## Description

### FIELD OF THE INVENTION

The present invention relates to a formulation having improved stability/ efficacy. The formulation is particularly suitable for treatment of various disorders. The invention also relates to a method of producing the formulation.

### BACKGROUND TO THE INVENTION

WO 2006/021814 describes a serum composition comprising corticotropin releasing factor (CRF).

WO 2006/021814 also describes the use of CRF for treating a number of disorders, in particular multiple sclerosis and inflammatory disorders such as rheumatoid arthritis; optic neuritis; motor neuron disease; autoimmune diseases; axonal or nerve damage; and cancers. Particular cancers of interest include myelomas, melanomas and lymphomas. Other disorders include cardiovascular diseases; and neural disorders, both demyelinating and non-demyelinating. Examples of particular disorders which may be treated with CRF include cerebrovascular ischemic disease; Alzheimer's disease; Huntingdon's chorea; mixed connective tissue diseases; scleroderma; anaphylaxis; septic shock; carditis and endocarditis; wound healing; contact dermatitis; occupational lung diseases; glomerulonephritis; transplant rejection; temporal arteritis; vasculitic diseases; hepatitis; and burns. Particular non-demyelinating disorders which may be treated include multiple system atrophy; epilepsy; muscular dystrophy; schizophrenia; bipolar disorder; depression; channelopathies; myasthenia gravis; pain due to malignant neoplasia; chronic fatigue syndrome; fibromyositis; irritable bowel syndrome; work related upper limb disorder; cluster headache; migraine; and chronic daily headache. Particular demyelinating disorders which may be treated include infections of the nervous system; nerve entrapment and focal injury; traumatic spinal cord injury; brachial plexopathy (idiopathic and traumatic, brachial neuritis, parsonage turner syndrome, neuralgic amyotrophy); radiculopathy; channelopathies; and tic douloureux. Particular autoimmune disorders which may be treated include lupus; psoriasis; eczema; thyroiditis; and polymyositis. Particular peripheral neuropathy of axonal and demyelinating type which may be treated include hereditary motor and sensor neuropathy of all types; Charcot-Marie-Tooth disease (CMT) types CMT1A, CMT1 B, CMT2, CMT3 (Dejerine Sottas disease), CMT4 (Types A, B, C and D), X-linked Charcot-Marie-Tooth disease (CMTX); Hereditary Neuropathy with liability to pressure palsies (HNPP), also called Tomaculous neuropathy; Hereditary Motor and Sensory Neuropathy with Deafness - Lom (HMSNL); Proximal Hereditary Motor and Sensory Neuropathy/Neuronopathy (HMSNP); Hereditary Neuralgic Amyotrophy; Hereditary Sensory and Autonomic Neuropathies (HSAN1, HSAN2, HSAN3 (also called Riley-Day syndrome or familial dysautonomia), HSAN4, HSAN5); Familial Amyloid polyneuropathies (Type I, Type II, Type III, Type IV); Metachromatic Leukodystrophy; Krabbe's Disease; Fabry's Disease; Adrenoleukodystrophy; Refsum's disease (HMSN IV); Tangier Disease; Friedreich's ataxia; Spinal cerebellar ataxia (SCA) all types - SCA1, SCA2, SCA3, SCA4, SCA5, SCA6, SCA7, SCA8, SCA10, SCA11, SCA12, SCA13, SCA14, SCA16; Spinocerebellar Ataxia; Cockayne's syndrome and Giant axonal neuropathy. CRF is also identified as being useful in the treatment of chronic inflammatory demyelinating polyneuropathy (CIDP) and Guillain-Barre syndrome. CRF is also described as having anti-angiogenic properties, caused by the molecules thrombospondin-1 (TSP-1) and platelet factor-4 (PF-4).

It is therefore understood that use of CRF, also known as corticotropin releasing hormone (CRH) also known as corticoliberin, is advantageous in treating the above-mentioned disorders in a patient.

One drawback associated with CRH formulations is that, following administration to a patient, the CRH protein has a limited effective biological half-life - by way of example, the CRH protein has a very low effective plasma half-life (approximately 4 minutes), and thus a low bioavailability/ efficacy, relying on a pulsatile stimulus.

Attempts to achieve long-term persistent levels of CRH *in vivo* have been reported, *e.g.* by Walker JJ *et al* (Walker et al (2012) "The origin of glucocorticoid hormone oscillations; PLoS Biol 10(6):e1001341). Even with constant infusion of CRH over a six-hour period, however, elevated levels of CRH can be maintained *in vivo* for no longer than an hour before falling back toward base-line levels; this effect appears to persist regardless of the dosage used. Moreover, constant infusion of CRH is undesirable and an impractical therapeutic method outside of a hospital or laboratory setting.

There is therefore a need for a CRH formulation having a longer effective biological half-life and thus a longer effective therapeutic window of efficacy.

Another drawback associated with CRH formulations is that said formulations have limited stability, and thus sub-optimal efficacy. There is therefore a need for a CRH formulation having improved stability and thus a longer effective therapeutic window of efficacy.

### SUMMARY OF THE INVENTION

The present invention addresses one or more of the above needs by providing a stabilised CRH formulation which demonstrates an extended effective biological half-life (e.g. persistence) in the body. Therapeutic uses of such stabilised CRH are also provided for prevention or treatment of one or more diseases (including, but not limited to, said hereinbefore listed disorders).

In more detail, the present invention provides a formulation comprising a stabilised complex of CRH and alpha-2 macroglobulin, wherein the latter is believed to protect CRH *in vivo.*

In one embodiment, the alpha-2 macroglobulin is present in the formulation of the invention at a concentration of more than 50,000 picograms per millilitre, for example between 100,000 and 150,000 picograms per millilitre, or between 110,000 and 130,000 picograms per millilitre. Thus, the present invention provides alpha-2 macroglobulin in a preferred amount for complexing with CRH.

Similarly, the CRH may be present in the formulation of the invention at a concentration of between 80 picograms per millilitre (pg/ml) and 120 pg/ml, for example between 90 and 110 pg/ml. Thus, the present invention provides CRH in an amount that is preferred for complexing with alpha-2 macroglobulin.

The alpha-2 macroglobulin may be present in the formulation of the invention at a concentration of between 100,000 and 150,000 picograms per millilitre, and the CRH may be present at a concentration of between 80 picograms per millilitre (pg/ml) and 120 pg/ml, for example between 90 and 110 pg/ml. The alpha-2 macroglobulin may be present in the formulation of the invention at a concentration of between 110,000 and 130,000 picograms per millilitre, and the CRH may be present at a concentration of between 80 picograms per millilitre (pg/ml) and 120 pg/ml, for example between 90 and 110 pg/ml.

The formulation of the invention may further comprise CRH binding protein (CRH-BP). The CRH-BP may be present at a concentration of between 30 and 60 picograms per millilitre (pg/ml), for example between 40 and 50 pg/ml.

In one embodiment, the stabilised CRH formulation of the present invention is prepared by a method that comprises: providing isolated blood from an ungulate (*e.g.* a goat), wherein said ungulate has been immunised with an autoimmune virus, and obtaining serum from the blood (*e.g.* by centrifugation); treating the serum to separate the CRH and other active components of interest; diafiltration of the separated serum comprising CRH and other active components of interest, thereby retaining molecules having a molecular weight of at least 10 KDa; filtering to remove molecules having a size greater than 0.2 microns; processing by nanofiltration to remove molecules having a size greater than 35 nanometres; wherein all of the above steps are performed under cooled conditions (*e.g.* less than 22 degrees C, preferably less than 10 degrees C, preferably less than 5 degrees C); whilst ensuring that the serum remains unfrozen following treatment to separate the CRH and other active components. As a final step, the processed serum is aliquoted into vials, optionally with protein concentration adjustment, to provide a single dose amount. At this stage it may be frozen (*e.g.* at minus 22 degrees C) prior to use. In this regard, prior to use, the aliquoted serum is thawed, followed by prompt administration (*e.g.* within 6 hours, preferably within 4 hours, preferably within 1 hour, preferably within 5 minutes, preferably within 1 minute) to a patient.

The autoimmune virus may be HIV or SIV, which may be HIV 3b; the autoimmune virus may be in the form of a lysate or a heat-killed virus.

In this regard, "serum" is defined as that component of the blood from which the blood cells have been removed, *e.g.* by centrifugation.

Thus, the basic methodology as described in WO 2006/021814, which is hereby incorporated in its entirety by reference thereto, may be employed, though with the inclusion of one or more additional steps selected from (i) a nanofiltration step; (ii) avoidance of multiple freeze-thaw steps and/ or minimising ambient temperature exposure; and/ or (iii) a mixing/ agitation step (to enhance CRH: alpha-2 macroglobulin complex formation).

Accordingly, the present invention provides a method of manufacturing a stabilised complex of CRH and alpha-2 macroglobulin, the method comprising:
(a) providing hyperimmune serum from an ungulate that has been immunised with an immunodeficiency virus;
(b) subjecting said hyperimmune serum to a microfiltration step that removes molecules having a size greater than 0.2 microns, thereby providing a micro-filtered serum;
(c) subjecting said micro-filtered serum to a nanofiltration step that removes molecules having a size greater than 35 nanometres, thereby providing a nano-filtered serum;
(d) aliquoting said nano-filtered serum into a vial, wherein said nano-filtered serum is in a form for administration to a patient, comprises the stabilised complex of CRH and alpha-2 macroglobulin, and wherein the serum remains unfrozen throughout steps (b) to (d).

Said microfiltration step may follow an ammonium sulphate precipitation plus (PBS) buffer dialysis step to retain molecules of, for example, at least 10 KDa. Other means of separating the CRH and other components of interest are also contemplated, which are well within the means of the person skilled in the art (separating columns, etc.).

Said nanofiltration step may be carried out using a 35 nanometre filter, which may be a 35 nanometre hollow fibre filter.

Optionally, exposure to ambient temperature (e.g. room temperature, 22 degrees C) is strictly minimised at all stages of the method - by way of example, cold trays (ensuring a maximum temperature of less than 22 degrees C, or less than 10 degrees C, or less than 7 degrees C, or less than 5 degrees C) and other such means are used throughout. Thus the composition may be kept at a constant temperature below 22 degrees C, e.g. at less than 10 degrees C, or less than 7 degrees C, or less than 5 degrees C. It is preferred that the method is carried out as a continuous process, avoiding any freezing steps prior to storage. For example, no freezing step is employed between the filtration step (*e.g.* micro- and/or nanofiltration) and final aliquoting into vials (optionally with adjustment of protein concentration). Most especially, it is preferred that no freezing step is carried out once the serum has been treated to separate the CRH and other components of interest, *e.g.* using ammonium sulphate.

One or more agitation steps may be carried out during the method; these agitation steps may use cold trays.

The method may comprise a final step (*e.g.* after the micro-/ nano-filtration step and any protein concentration adjustment step) of freezing for subsequent storage. In one embodiment, the hyperimmune serum is not frozen prior to micro- and/or nano-filtration.

Without wishing to be bound by any theory, the present inventors believe that the bioactive molecules within the composition comprising CRH are prone to undesirable aggregation upon freezing and thus this step should not be performed more than once prior to use. Thus, multiple freezing-thawing steps should be avoided as they are believed to result in inactivation of key bioactive molecules within the CRH composition and/or lead to removal thereof during subsequent filtration.

Alternatively, the CRH formulation of the present invention may be prepared from first principles based on commercially available components (including recombinantly prepared components).

The two principal components of the hereindescribed formulation (when present in the stabilised complex form) may have a ratio of 4:1 CRH:alpha-2 macroglobulin, 2:1 CRH:alpha-2 macroglobulin, 1:1 CRH:alpha-2 macroglobulin, or combinations thereof. In one embodiment, the predominant complexed form of CRH: alpha-2 macroglobulin is a macromolecular quaternary complex (i.e. 4:1).

The CRH and alpha-2 macroglobulin components may be complexed together via non-covalent bonds such as one or more of hydrogen bonds, ionic bonds, van der Waals forces, and hydrophobic interactions.

The above-described concentrations typically refer to the concentrations obtained during the manufacture process such as immediately prior aliquoting and optional freezing for subsequent storage (optionally including any protein concentration adjustment) that yields the ready-to-use formulation (typically having a concentration of 4-5 milligrams protein per millilitre).

The CRH may be human or non-human. For example, the CRH may be an ungulate CRH such as horse, zebra, donkey, cattle, bison, goat, pig, moose, elk, deer, antelope or gazelle. In one embodiment the CRH is not ovine CRH. In a most preferred embodiment, the CRH is caprine CRH.

Corticotropin releasing hormone (CRH), also known as corticoliberin, is a 41 residue peptide originally isolated from ovine hypothalamus based on its ability to stimulate the hypothalamic-pituitary adrenal axis from cultured anterior pituitary cells. CRH is the principal neuroregulator of the basal and stress-induced secretion of ACTH, β-endorphin, and other pro-opiomelanocortin related peptides from the paraventricular nucleus of the anterior pituitary gland. In addition to its endocrine function, mediation of CRH specific responses occur via its cognate receptors (they include CRH-R1, CRH-R2α, CRH-R2β, CRH-R2γ and CRH-binding protein [CRH-BP]). CRH-R1 is a 415 amino acid protein that shows sequence homology across different species (human, mouse and rat). CRH-binding protein represents the smallest receptor at 322 amino acids, and acts as an inhibitor of free CRH. CRH-R1 and CRH-R2 are both ubiquitously expressed on the cell surface of the hypothalamus, cerebellum, cortex, amygdala, subcortex, immune cells, gut and skin. Conversely, CRH-BP is found predominantly in the liver, placenta and brain. Importantly there appears to be no significant overlap in distribution of the said receptors. This likely reflects differing functional roles. An example of this is seen during pregnancy were elevation in peripheral CRH is regulated by an elevation in secreted levels of CRH binding protein. The overall effect of this is to prevent an elevation in peripheral circulating levels of glucocorticoids during pregnancy. Several forms of CRH have been identified in nature, they include a high molecular weight form 194 amino acids *Mw ~* 30,000, a *Mw* -18,000, *Mw* ~7,500 and the 41 amino acid residue. All three forms are biologically active and able to stimulate ACTH release.

Alpha-2-macroglobulin, also known as a2M, is a large plasma protein found in blood. It is produced by the liver and is the largest major non-immunoglobulin protein in plasma. A2M is synthesized primarily by the liver and is also produced locally by macrophages fibroblasts and adrenocortical cells. Alpha-2-macroglobulin acts as an anti-protease and is able to inactivate an enormous variety of proteinases. It also functions as a carrier protein binding to numerous growth factors and cytokines. Examples include transferrin (where a2M regulates the binding of to the surface receptor), binds defensin and myelin basic protein, binds several important cytokines, including basic fibroblast growth factor (bFGF), platelet derived growth factor (PDGF), nerve growth factor (NGF), interleukin-1β (IL-1β) and interleukin-6 (IL-6), transforming growth factor (TGF-1β), and insulin, and modify their biological activity. Human a2M is composed of four identical subunits bound together by-S-S-bonds. The principal mechanism by which a2M inhibits proteases is through steric hindrance. The mechanism involves protease cleavage of the thiol 35 amino acid bait region, a segment of the molecule, which is particularly susceptible to proteolytic cleavage, which initiates conformational change such that a2M collapses about the protease thus resulting in its inhibition. In the resulting a2M-protease complex, the active site of the protease is sterically shielded, thus substantially decreasing access to protein substrates including those that are bound to active a2M. Decreases in a2M been associated with a variety of diseases, or example common variant (29.5%) polymorphism of a2M needs to increase the risk of Alzheimer's disease.

Stabilisation of CRH by binding to alpha-2 macroglobulin, and the attendant enhanced effective biological half-life is one effect of formulations of the present invention. Said stability provides an enhanced (longer) plasma half-life - by way of example, the stabilised complex of the present invention has a half-life of at least 24 hours.

The stabilised complex of the invention may be employed in combination with one or more other stabilisers, such as fibronectin or albumin. Additionally or alternatively, the stabilised complex of the invention may be employed in combination with a pro-opiomelanocortin (POMC) peptide. The use of POMC peptides in this manner stimulates further *in vivo* POMC production, and/ or helps to induce a host response before endogenous POMC peptide levels are stimulated.

POMC may be present in the formulation at a range of between 140 picomoles per litre (pmol/l) and 200 pmol/l.

Thus, in one embodiment the invention provides a stabilised CRH formulation having component proportions as defined herein. For example, the formulation may have between 110,000 and 130,000 picograms per millilitre (pg/ml) of alpha-2 macroglobulin, between 60 pg/ml and 120 pg/ml of CRH, and optionally between 140 pmol/l and 200 pmol/l of POMC.

Without wishing to be bound by any theory, the present inventors believe that alpha-2 macroglobulin inhibits subtilisin serine endopeptidases (pro-hormone convertases), which may otherwise exert deleterious effects on POMC prior to administration.

Following administration, *in vivo* activation of POMC may occur by the direct actions of prohormone convertase 1 (PC1), prohormone convertase 2 (PC2), carboxypeptidase E (CPE), peptidyl alpha-amidating monooxygenase (PAM), N-acetyltransferase (N-AT) and prolylcarboxypeptidase (PRCP) acting in a tissue specific manner. All said POMC cleavage sites appear to be acted upon by proteases in the hypothalamus, placenta, epithelium and leucocytes.

Human POMC peptide is described in detail in entry 176830 of OMIM (online mendelian inheritance in man, accessible through http://www.ncbi.nlm.nih.qovA). The nucleotide and amino acid sequence of human POMC is also known, and has GENBANK accession number BC065832. Human POMC gives rise to a glycosylated protein precursor having a molecular weight of 31 kDa.

By "a POMC peptide" is meant any peptide having a corresponding sequence, structure, or function. It will be apparent to the skilled person that the canonical nucleotide and/or amino acid sequences given for human POMC in the GENBANK entry referenced above may be varied to a certain degree without affecting the structure or function of the peptide. In particular, allelic variants and functional mutants are included within this definition. Mutants may include conservative amino acid substitutions. "A POMC peptide" as used herein refers to any peptide acting as a precursor to at least one form of MSH, ACTH, at least one form of lipotrophin (LPH), β endorphin, met-enkephalin and leu-enkephalin; and preferably all of α, β, and γ MSH; ACTH; β and γ LPH; and β endorphin, met- enkephalin and leu-enkephalin.

The POMC peptide may be human or non-human POMC. In one embodiment the POMC peptide is an ungulate POMC such as horse, zebra, donkey, cattle, bison, goat, pig, moose, elk, deer, antelope or gazelle. In one embodiment the POMC peptide is not a rodent (e.g. mouse or rat) POMC peptide.

Administration of a POMC peptide has a self-sustaining effect, in that administration of an initial amount of POMC peptide leads to endogenous production of POMC in the patient; thus, an initial administration of a low level of POMC has a significant effect on the patient.

The formulation of the invention selectively increases the enzymatic degradation of POMC *in vivo.* The formulation of the invention increases the release of POMC-derived peptides such as ACTH, alpha-MSH, beta-MSH, CLIP, Lipotrophin-gamma, met-enkephalins and beta-endorphins. Longer-term administration of the formulation typically leads to a sustainable increase in POMC-derived peptides in a patient.

The formulation of the invention typically possesses a reduced amount of immunoglobulin component. Thus, the associated method of the present invention provides a formulation in which the immunoglobulin component has been minimised - for example, in said formulation, the immunoglobulin component has been minimised so that the formulation contains less than 4.5 mg/ml, for example less than 4 mg/ml or less than 3.9 mg/ml. A reduced immunoglobulin component is preferred as this helps to minimise a host immune response against said component (notably against any IgG component). The above-described concentrations typically refer to the concentrations obtained during the manufacture process such as immediately prior to aliquoting and optional freezing for subsequent storage (optionally including any protein concentration adjustment) that yields the ready-to-use formulation.

The stabilised complex of the invention acts to enhance the central CRH-1 regulatory response. This results in optimisation of key anti-inflammatory cytokines and abrogation of certain pro-inflammatory cytokines related to the Th1-mediated response.

Administration of CRH to a patient stimulates production of endogenous CRH and thus leads to a self-sustaining effect. CRH can therefore be administered at a low concentration to a patient.

The stabilised complex of the invention also protects CRH from proteolytic degradation by proteases and accordingly administration of the stabilised complex of the invention provides slow release of CRH in the circulation and a significant increase in CRH levels.

Thus, the stabilised complex of the invention leads to persistent, elevated levels of CRH *in vivo* for at least 12 hours following administration, for at least 24 hours following administration, or for at least 48 hours following administration. Administration of the stabilised complex of the invention may lead to an *in vivo* increase in CRH concentration of between 25% and 50% from patient baseline CRH levels at 24 hours from administration, and an *in vivo* increase of between 75% and 100% from patient baseline CRH levels at 48 hours from administration.

This contrasts with administration of formulations containing CRH in similar concentrations where the CRH is not in a stabilised complex (or in a poorer stabilised complex including, for example CRH aggregates): an *in vivo* increase in CRH concentration from patient baseline CRH levels may be seen within 24 hours of administration, but the effect does not persist and within 36 hours from administration CRH concentration is falling.

The formulation of the invention selectively up-regulates the CRH-1 receptor both centrally and peripherally in key target issues and enhances the central CRH-1 regulatory response, while also leading to selective down-regulation of CRH-2 specific receptors and up-regulation of CRH-binding protein in tissues such as the adrenal cortex. It is believed by the inventors that administration of the formulation will thus lead to optimisation of key inflammatory cytokines and down-regulation of certain pro-inflammatory cytokines related to the Th-1 mediated response, through targeting leucocytes and macrophages.

No secondary glucocorticoid surge from increased levels of ACTH are noted despite the elevated CRH levels, in part due to further processing and cleavage of ACTH and a reduction in "free" CRH levels. Elevated "free" peripheral CRH may also be regulated by a parallel increase in CRH-BP. This again mitigates excessive production of unwanted glucocorticoids. The combination of the peripheral and central dynamic relationship provides a prolonged activation of the CRH pathway without negative feedback. Central CRH is then able to further target T-helper immune cells and macrophages that express CRH.

The combined net effect of all of the above is an anti-inflammatory, reparative and fundamental immunomodulatory therapeutic that works within homeostatic constraints. The unique targeting and accessibility of the stabilised complex of the invention explains its versatility in a wide range of diseases.

Thus the present invention provides a stabilised CRH formulation that works within homeostatic constraints following *in vivo* administration.

The present invention accordingly further provides a formulation for use in prevention or treatment of one or more diseases selected from systemic sclerosis (SSc), multiple sclerosis and inflammatory disorders such as rheumatoid arthritis; optic neuritis; motor neuron disease; autoimmune diseases; axonal or nerve damage; and cancers (including myelomas, melanomas and lymphomas); cardiovascular diseases; neural disorders, both demyelinating and non-demyelinating; cerebrovascular ischemic disease; Alzheimer's disease; Parkinson's disease; Huntingdon's chorea; mixed connective tissue diseases; scleroderma; anaphylaxis; septic shock; carditis and endocarditis; wound healing; contact dermatitis; occupational lung diseases; glomerulonephritis; transplant rejection; temporal arteritis; vasculitic diseases; hepatitis (in particular hepatitis C); burns; multiple system atrophy; epilepsy; muscular dystrophy; schizophrenia; bipolar disorder; depression; channelopathies; myasthenia gravis; pain due to malignant neoplasia; chronic fatigue syndrome; fibromyositis; irritable bowel syndrome; work related upper limb disorder; cluster headache; migraine; chronic daily headache; infections of the nervous system; nerve entrapment and focal injury; traumatic spinal cord injury; brachial plexopathy (idiopathic and traumatic, brachial neuritis, parsonage turner syndrome, neuralgic amyotrophy); radiculopathy; channelopathies; tic douloureux; lupus; psoriasis; eczema; thyroiditis; polymysotis; hereditary motor and sensor neuropathy of all types; Charcot-Marie-Tooth disease (CMT) types CMT1A, CMT1 B, CMT2, CMT3 (Dejerine Sottas disease), CMT4 (Types A, B, C and D), X-linked Charcot-Marie-Tooth disease (CMTX); Hereditary Neuropathy with liability to pressure palsies (HNPP), also called Tomaculous neuropathy; Hereditary Motor and Sensory Neuropathy with Deafness - Lom (HMSNL); Proximal Hereditary Motor and Sensory Neuropathy/Neuronopathy (HMSNP); Hereditary Neuralgic Amyotrophy; Hereditary Sensory and Autonomic Neuropathies (HSAN1, HSAN2, HSAN3 (also called Riley-Day syndrome or familial dysautonomia), HSAN4, HSAN5); Familial Amyloid polyneuropathies (Type I, Type II, Type III, Type IV); Metachromatic Leukodystrophy; Krabbe's Disease; Fabry's Disease; Adrenoleukodystrophy; Refsum's disease (HMSN IV); Tangier Disease; Friedreich's ataxia; Spinal cerebellar ataxia (SCA) all types - SCA1, SCA2, SCA3, SCA4, SCA5, SCA6, SCA7, SCA8, SCA10, SCA11, SCA12, SCA13, SCA14, SCA16; Spinocerebellar Ataxia; Cockayne's syndrome; Giant axonal neuropathy; chronic inflammatory demyelinating polyneuropathy (CIDP); and Guillain-Barre syndrome.

The formulation of the present invention may take the form of a pharmaceutical composition. The invention accordingly provides a pharmaceutical composition comprising the stabilised complex of the invention, and the use thereof in preventing or treating of one or more of the above-mentioned diseases.

Administration of the formulation of the invention may be accomplished orally or parenterally. Methods of parenteral delivery include topical, intra-arterial, intramuscular, subcutaneous, intramedullary, intrathecal, intra-ventricular, intravenous, intraperitoneal, or intranasal administration.

In addition to the active ingredients, the formulation of the invention may comprise suitable pharmaceutically acceptable carriers comprising excipients and other components which facilitate processing of the active compounds into preparations suitable for pharmaceutical administration.

Oral formulations may include pharmaceutically acceptable carriers known in the art in dosages suitable for oral administration. Such carriers enable the compositions to be formulated as tablets, pills, dragees, capsules, liquids, gels, syrups, slurries, suspensions, and the like suitable for ingestion by the subject.

Formulation for oral use can be obtained through combination of active compounds with a solid excipient, optionally grinding a resulting mixture, and processing the mixture of granules, after adding suitable additional compounds if desired to obtain tablets or dragee cores. Suitable excipients include carbohydrate or protein fillers such as sugars, including lactose, sucrose, mannitol, sorbitol; starch from corn, wheat, rice, potato, or other plants; cellulose such as methylceilulose, hydroxypropylmethylcellulose, or sodium carboxymethylcellulose; and gums including arabic and tragacanth; as well as proteins such as gelatin and collagen. If desired, disintegrating or solubilising agents may be added, such as cross linked polyvinyl pyrrolidone, agar, alginic acid, or a salt thereof.

Dragee cores can be provided with suitable coatings such as concentrated sugar solutions, which may also contain gum arabic, talc, polyvinyl pyrrolidone, carbopol gel, polyethylene glycol, titanium dioxide, lacquer solutions, and suitable organic solvents or solvent mixtures. Dyestuffs or pigments may be added to the tablets or dragee coatings for product identification or to characterise the quantity of active compound.

Formulations for oral use include push-fit capsules made of gelatin, as well as soft, sealed capsules made of gelatin and a coating such as glycerol or sorbitol. Push-fit capsules can contain active ingredients mixed with a filler or binders such as lactose or starches, lubricants such as talc or magnesium stearate, and, optionally stabilisers. In soft capsules, the active compounds can be dissolved or suspended in suitable liquids, such as fatty oils, liquid paraffin, or liquid polyethylene glycol with or without stabilisers.

Formulations for parenteral administration include aqueous solutions of active compounds. For injection, the formulations of the invention may take the form of aqueous solutions, preferably in physiologically compatible buffers such as Hank's solution, Ringer's solution, or physiologically buffered saline. Aqueous suspension injections can contain substances which increase the viscosity of the suspension, such as sodium carboxymethyl cellulose, sorbitol, or dextran. Additionally, suspensions of the active compounds can be prepared as appropriate oily injection suspensions. Suitable lipophilic solvents or vehicles include fatty oils such as sesame oil, or synthetic fatty acid esters, such as ethyl oleate or triglycerides, or liposomes. Optionally, the suspension can also contain suitable stabilisers or agents which increase the solubility of the compounds to allow for the preparation of highly concentrated solutions.

For topical or nasal administration, penetrants appropriate to the particular barrier to be permeated may be used in the formulation.

The formulations of the present invention can be manufactured substantially in accordance with standard manufacturing procedures known in the art. The formulation may also comprise one or more peptide regulatory or releasing factors, which may induce a cascade of release of further peptides by a variety of cells in the patient. Such additional factors are typically provided from the same animal species as the CRH. Suitable factors include α- HLA, TGF-β, and IL-10, among others.

In one embodiment, the formulation may comprise one or more of vasopressin, beta endorphin, and an enkephalin. In certain embodiments, the formulation may comprise CRH binding protein, CRH-BP. This binds CRH and acts as a reservoir for subsequent release of CRH to the patient.

The present invention also provides a formulation for use in a method of treatment for a disease selected from systemic sclerosis (SSc); multiple sclerosis; rheumatoid arthritis; optic neuritis; Parkinson's disease; motor neuron disease; autoimmune diseases including lupus, psoriasis, eczema, thyroiditis, and polymyositis; axonal or nerve damage; cancers, in particular myelomas, melanomas, and lymphomas; neural disorders, both demyelinating and non-demyelinating; inflammatory conditions; obesity; nerve conduction disorders; and sexual dysfunction, in particular erectile dysfunction; the method comprising administering the formulation of the invention to a patient in need thereof.

The optimal dosage will be determined by the clinician. For example, administration may be in a dosage of between 0.01 and 10 mg (total protein) per kg (patient), for example between 0.01 and 5 mg/kg, between 0.025 and 2 mg/kg, or between 0.05 and 1 mg/kg. A product suitable for administration to patients may have a total protein concentration of approximately 4 mg/ml.

The precise dosage to be administered may be varied depending on such factors as the age, sex and weight of the patient, the method and formulation of administration, as well as the nature and severity of the disorder to be treated. Other factors such as diet, time of administration, condition of the patient, drug combinations, and reaction sensitivity may be taken into account. An effective treatment regimen may be determined by the clinician responsible for the treatment. One or more administrations may be given, and typically the benefits are observed after a series of at least three, five, or more administrations. Repeated administration may be desirable to maintain the beneficial effects of the composition.

The treatment may be administered by any effective route, such as by subcutaneous injection, although alternative routes which may be used include intramuscular or intra-lesional injection, oral, aerosol, parenteral, topical or via a suppository.

The treatment may be administered as a liquid formulation, although other formulations may be used. For example, the treatment may be mixed with suitable pharmaceutically acceptable carriers, and may be formulated as solids (tablets, pills, capsules, granules, etc) in a suitable composition for oral, topical or parenteral administration.

The invention also provides use of the aforementioned formulation in the preparation of a medicament for the treatment of one or more of the diseases recited above.

### BRIEF DESCRIPTION OF THE DRAWINGS

These and other aspects of the present invention will now be described by way of example only with reference to the accompanying drawings, in which:
Figures 1 and 2 show *in vivo* change in CRH concentrations in pg/ml (Figure 1A to 1C) and *in vivo* change in CRH binding protein concentrations (Figure 2A to 2C) in mice treated with either the composition of the invention "Aimspro" (1A, 2A), naïve serum - though otherwise prepared by exactly the same manufacture protocol as the present invention "Naïve serum" (1 B, 2B), or CRH formulation prepared by the present applicant's prior art manufacture protocol "Prior art CRH formulation" (1C, 2C);
Figures 3 and 4 show mass spectrometry analyses of patient sera before and after treatment with the stabilised complex of the invention;
Figures 5 and 6 show *in vivo* activity of the stabilised complex of the invention following treatment;
Figures 7 to 10 show evidence for a switch in inflammatory profile of patients following treatment with the stabilised complex of the invention;
Figure 11 shows levels of CRH in human serum and the stabilised complex of the invention;
Figure 12 shows a summary statistical table of absolute CRH levels and changes relative to baseline (picograms per millilitre) in patients with diffuse systemic sclerosis, measured at baseline and at 26 weeks, following a twice-weekly treatment with either the composition of the invention or placebo (controls);
Figure 13 shows a plot of the difference between the baseline (pre-treatment) level of CRH and the final level of CRH in patients with diffuse systemic sclerosis, measured at baseline and at 26 weeks, following a twice-weekly treatment with either the composition of the invention or placebo;
Figure 14 shows a summary statistical table of absolute CRH levels (nanograms per millilitre) in patients with secondary progressive multiple sclerosis, measured at baseline and at 4 weeks following a twice-weekly treatment with either the composition of the invention or placebo (controls).

### EXAMPLES

CRH and products containing CRH are very sensitive to proteolytic degradation and suffer from the above-mentioned poor effective half-life following *in vivo* administration. We show here that the stabilized complex of the invention has improved *in vivo* stability and, in view of said enhanced biological half-life, is able to demonstrate a greater therapeutic efficacy.

### Example 1: Manufacture of the stabilised complex of the invention

Hyperimmune ungulate serum is centrifuged to separate any unwanted components, and the method carried out as a continuous process, avoiding any freezing or thawing step(s) prior to final aliquoting. This avoids any aggregation and loss of the CRH component from the formulation.

In more detail, a serum composition comprising CRH was stored at 2 to 8 degrees C (and not frozen) and was diluted at a ratio of 1:2 parts cold PBS, and supersaturated ammonium sulphate was added slowly with constant agitation until a ratio of 47:53 of ammonium sulphate:PBS was reached. This was carried out on a cold tray and the resulting solution was maintained at this temperature for 30 to 60 minutes with constant agitation.

The serum solution was then centrifuged in a Beckman J6M/E centrifuge at 3500 rpm for 45 minutes at 4 degrees C. The supernatant was removed and discarded. The precipitated solid material was re-suspended in cold 50% saturated ammonium sulphate:PBS solution and re-centrifuged at 3500 rpm at 4 degrees C for 45 minutes. The supernatant was again discarded and the precipitated solid material re-suspended in ice cold PBS buffer. This solution (the serum component) was then subjected to diafiltration at 4 degrees C against PBS with a molecular weight cut-off of 10,000 Daltons.

A two-stage filtration step was then carried out, whereby the solution was filtered through a 0.2 micrometre filter and was then passed through a 35 nanometre hollow fibre filter. Following the filtration step, the solution was adjusted to a protein concentration of between 4 to 5 milligrams per millilitre with ice-cold PBS.

Small batches of the solution (1.1 millilitres) containing the stabilised composition of the invention were put into vials in an isolator. 1 millilitre single doses were thus obtained and stored at -15 to -25 degrees C prior to use.

All steps were carried out as a continuous process in the cold except nanofiltration and filling into vials, which were both conducted so as to minimise exposure to ambient temperature at all times. Cold trays were used whenever possible. Following treatment of the serum with PBS and supersaturated ammonium sulphate, freezing was avoided until the final filling stage and subsequent storage.

### Example 2: The stabilised complex provides a persistent, elevated concentration of CRH in vivo

The stabilised complex of the invention has been compared with prior art formulations as previously disclosed by applicant.

Applicant has disclosed the same basic manufacture protocols in WO 2003/004049, WO 2003/064472, WO 2005/056053, WO 2005/097183, WO 2006/021814, and WO 2007/077465.

In this Example, male mice, C57BL/6, ~25 gm were divided into three groups: one group was administered the stabilised complex of the invention ("Aimspro"); another group was administered a naive caprine serum (*i.e.* from a goat that had not been immunised) but which had been otherwise prepared by exactly the same manufacture (including 35 nanometre filtration step) method of the present invention ("Naïve serum"); and the third group was administered a composition comprising a CRH formulation prepared by Applicant's prior art basic manufacture protocol ("Prior art CRH formulation").

Samples of approximately 250µl of whole blood (retro-orbital) were collected via the retroorbital plexus using a microcapillary (EDTA) tube at intervals of 15 minutes, half an hour, 1 hour, 3 hours, 6 hours, 12 hours, 24 hours and 48 hours from initial administration. Li Heparin-whole blood was centrifuged at 4 degrees C for 10 minutes at a RCF of 1000 and stored at -80 degrees C until CRH determination was carried out by ELISA.

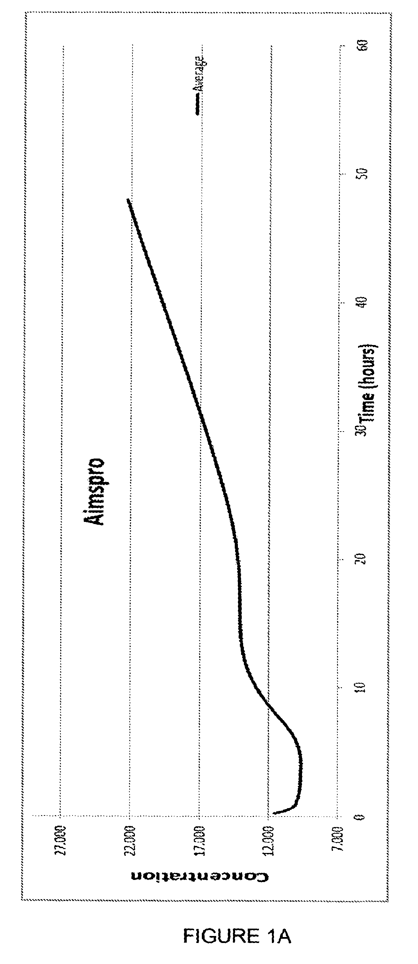

The average CRH concentration was calculated in respect of each population and the results are shown in Figure 1A through 1C.

It can be seen from Figure 1A that the composition of the invention (labelled "Aimspro") provided a steady, sustained increase in CRH concentration within the population over time. In particular, CRH concentration was still increasing at the 48-hour point.

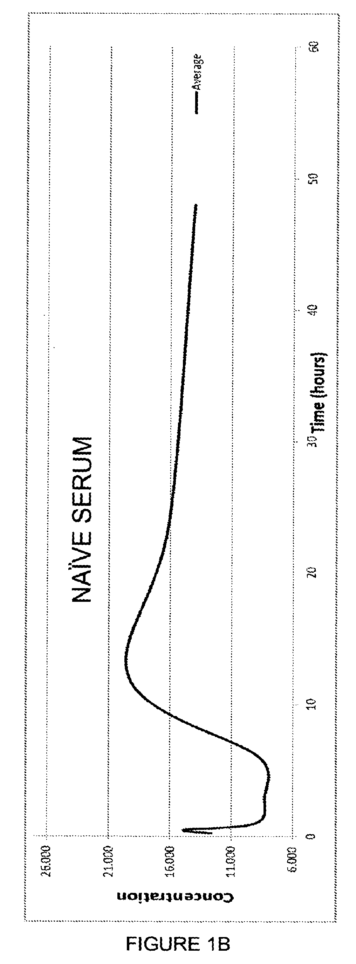

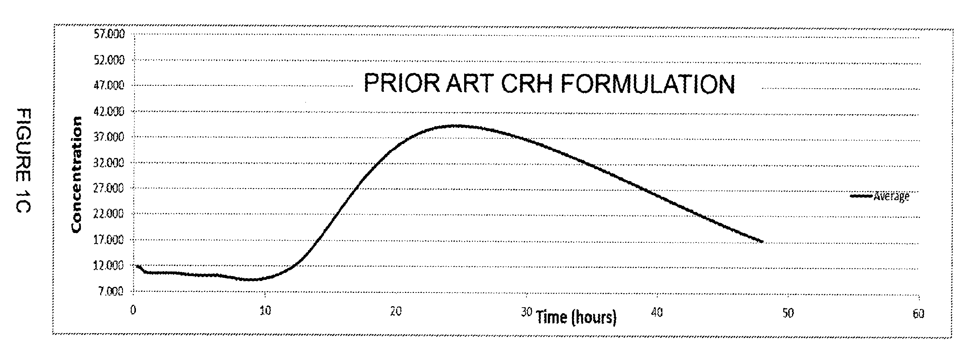

By contrast, the naïve serum ("Naïve serum" - Figure 1 B) and the prior art CRH formulation ("Prior art serum formulation" - Figure 1C) did not provide a steady expression profile and the level of CRH at the 48-hour point is clearly falling back to initial levels.

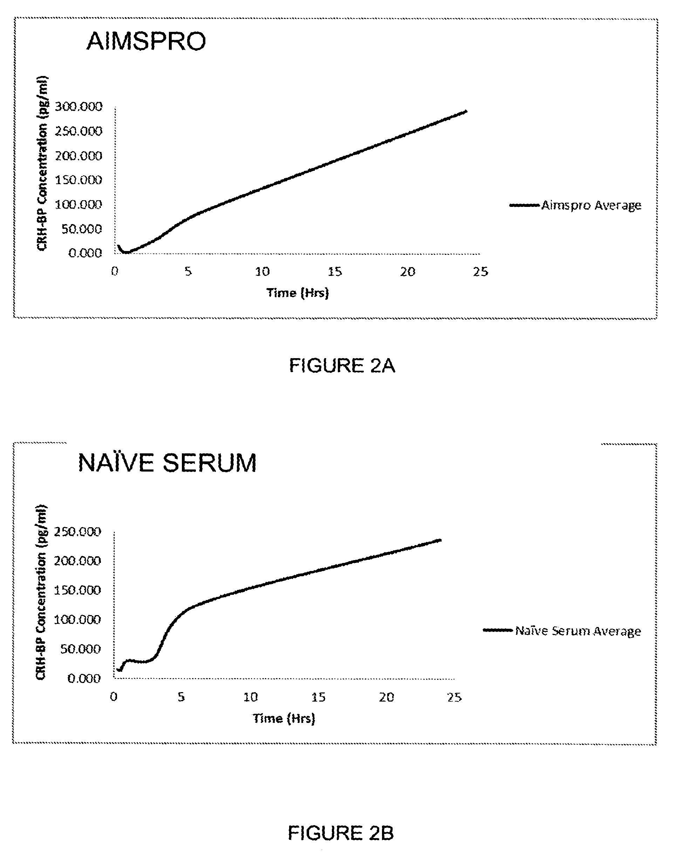

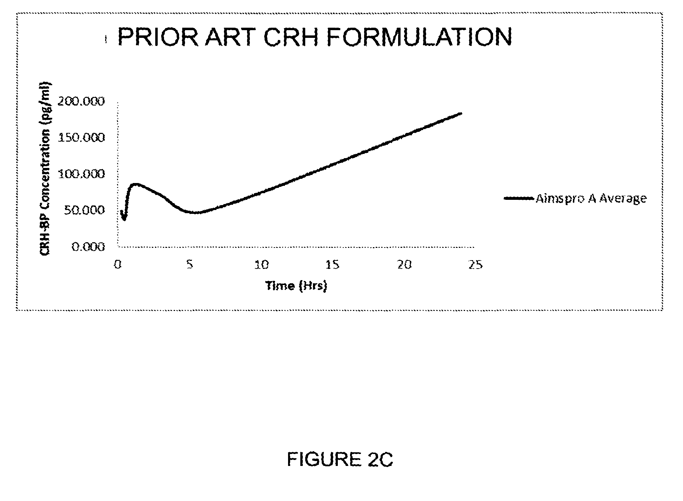

By way of comparison, the level of CRH binding protein (CRH-BP) was also measured and the average result was calculated for each population over time. The results are shown in Figure 2A through 2C. It can be seen that the expression profile is similar in each population.

The table below provides a comparison of the composition of the stabilised complex of the present invention ("Aimspro") against the composition of the prior art CRH formulation ("Prior art CRH formulation").

| COMPONENT | Aimspro | Prior art CRH formulation |
|---|---|---|
| CRH-BP (pg/ml) | 41.7 | 98.84 |
| a2M (pg/ml) | 122,014 | 35,738 |
| CRH half-life (hours) | 10.2 | 1 |

### Example 3: Treatment induces protein/peptide expression in patients' sera

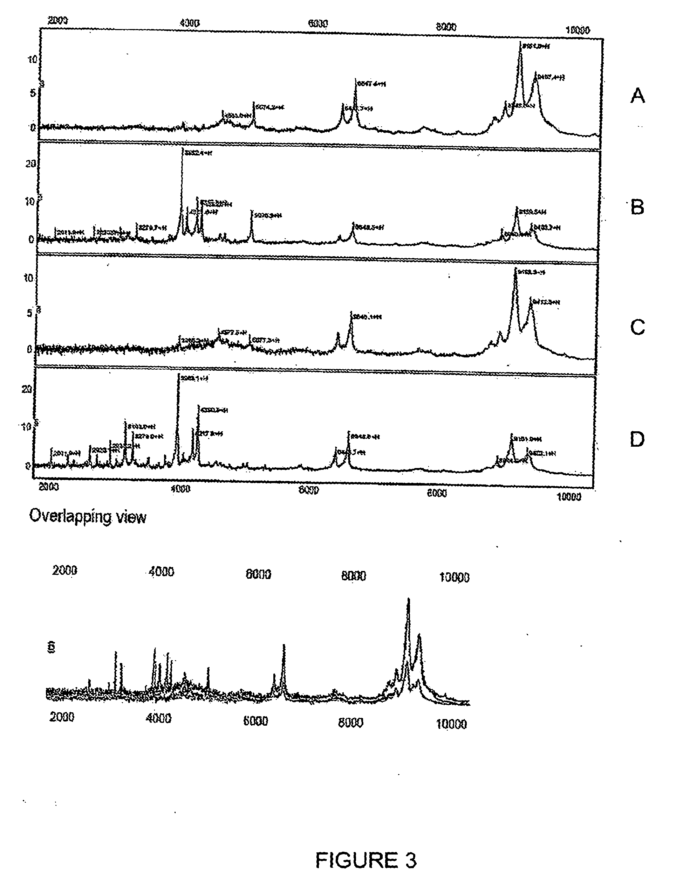

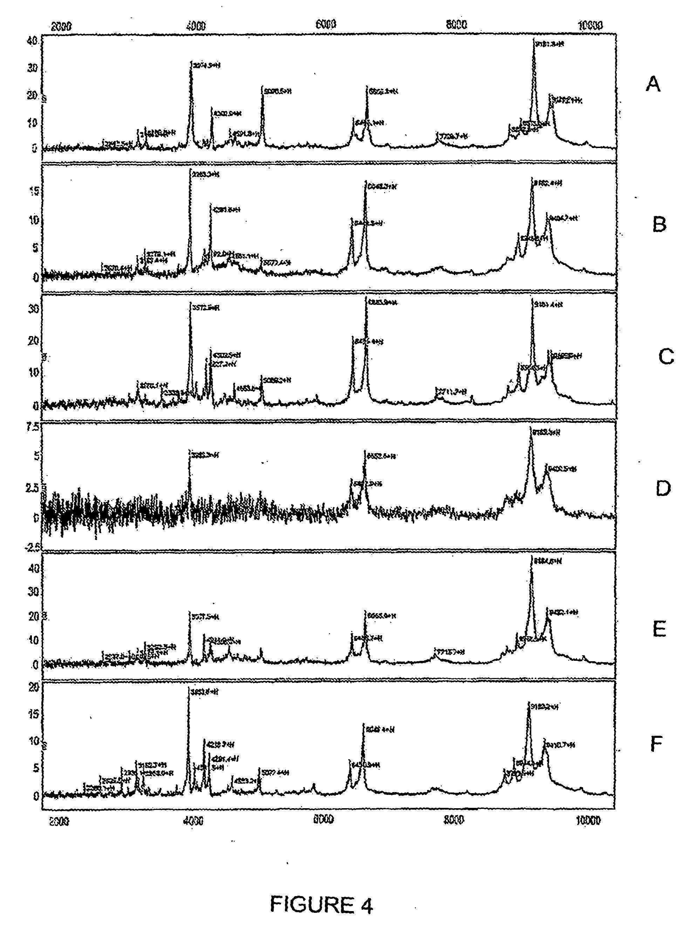

Mass spectrometry of patients' sera was carried out before and after treatment with the stabilised complex of the invention. The spectra from 2 kDa to 10 kDa are compared, as it is this molecular weight range which is associated with the bioactive peptides of interest discussed above. The results of serum analysis in a first patient are shown pre-treatment (Figure 3A) and post-treatment (Figure 3B). The results of serum analysis in a second patient are shown pre-treatment (Figure 3C) and post-treatment (Figure 3D). A comparison of the profiles in the pre- and post-treatment sera of both patients reveals clear differences in the peptide expression in the 2 kDa to 6 kDa region. For ease of comparison an overlapping view of the profiles is also provided (Figure 3E). A comparative peptide / protein expression in post-treatment sera of six treated patients is also provided (Figure 4A to Figure 4F). When compared to the pre-treatment expression of the two patients in Figure 3A and Figure 3C, it can be seen in from Figures 4A to 4F that each patient shows increased levels of induced peptide / protein expression particularly in the 4 kD region.

### Example 3: Evidence for the in vivo activity of the stabilised complex of the invention

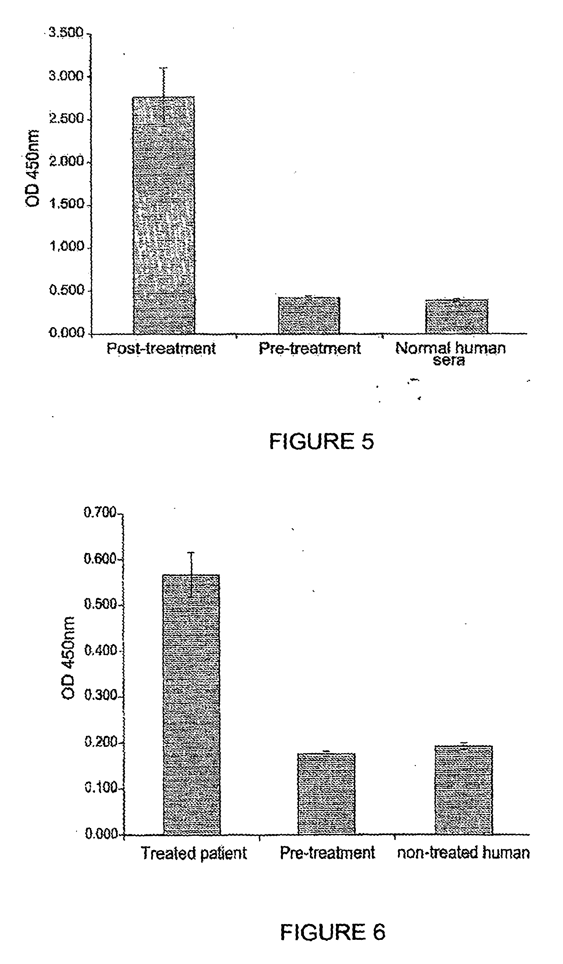

Figure 5 shows comparative levels of ACTH in the sera of patients before and after receiving treatment with the stabilised complex of the invention. This is also compared with levels of ACTH in the sera of healthy volunteers. Sera were diluted 1:100 and quantified by an ELISA of sera compared with the product. Data are the mean of three determinations +/- standard errors. Post treatment n=5; pre treatment n=3; normal human sera n=5. The data show that treatment with the stabilised complex of the invention increases ACTH levels. Figure 6 shows comparative levels of β endorphin in the serum of patients before and after receiving treatment with the *s*tabilised complex of the invention. This is compared with levels of β endorphin in the sera of healthy volunteers. Sera were diluted 1:100 and quantified by an ELISA of sera compared with the product. Data are the mean of three determinations +/standard errors. The data show that treatment increases β endorphin levels.

### Example 4: The stabilised complex of the invention leads to a change from pro-inflammatory TH-1 profile to an anti-inflammatory TH-2 cytokine profile in treated patients

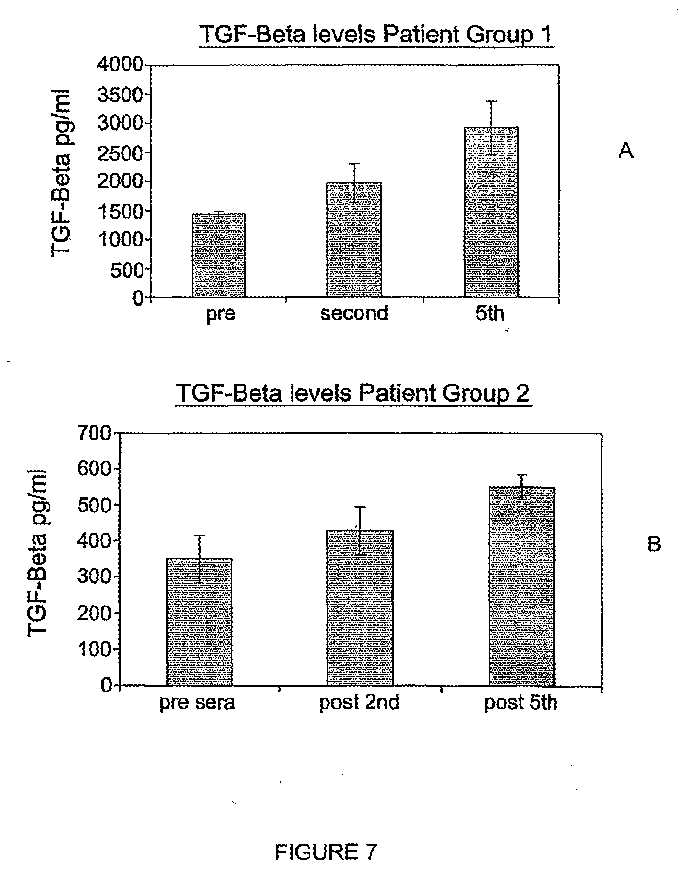

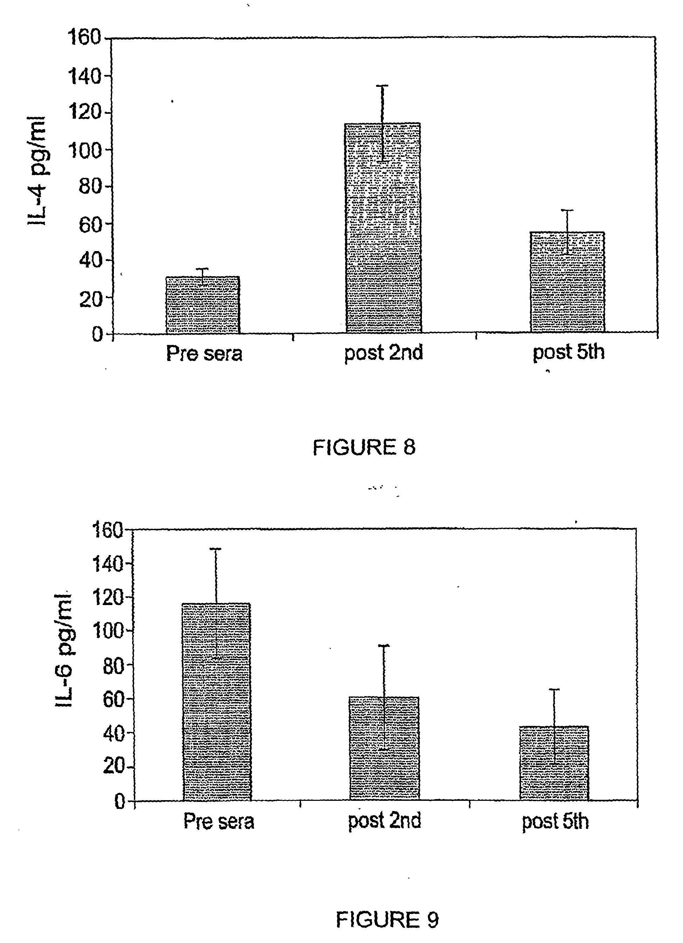

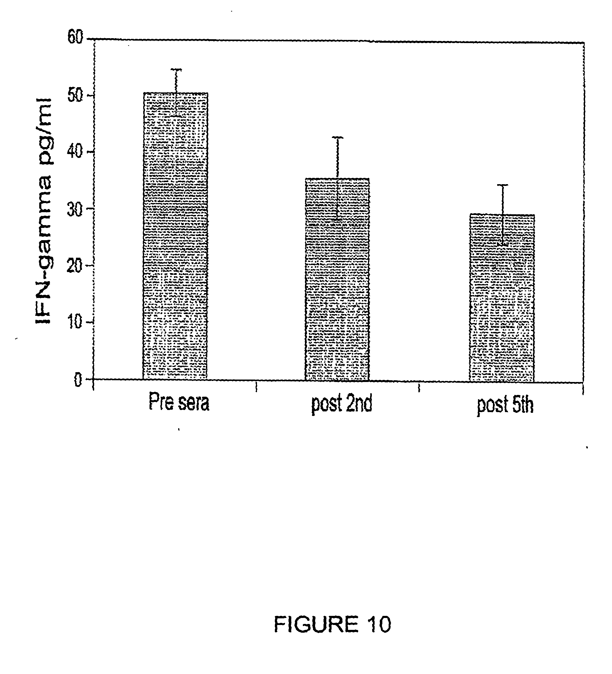

Figure 7 shows the levels of TGF-β in the serum of two groups of patients (group 1, shown in Figure 7A; and group 2, shown in Figure 7B) before and after treatment with the stabilised complex of the invention. The two groups of patients (n=3 for each group) show differing responses with respect to the concentrations of TGF-β produced, but all patients showed an increase in serum levels in response to treatment (pre sera = patients' serum levels before treatment; post 2nd and post 5th = after the 2nd and 5th administration). The data show that treatment with the stabilised complex of the invention induces increased concentration of the anti-inflammatory cytokine TGF-β. Figure 8 shows the levels of IL-4 in the serum of one group of patients before (pre-sera) and after treatment with the stabilised complex of the invention. It can be seen that after treatment (post 2nd), the levels of IL-4 are significantly increased in the patients' sera (n=5). However, following the 5th administration, the levels of IL-4 had dropped in all patients, but nevertheless remained higher than they had been pre-treatment. IL-4 is known to down-regulate the production of the pro-inflammatory cytokines from TH-I cells. It may be that the consistent changes in concentration seen in all patients are consistent with IL-4's role in the switch from TH-1 to TH-2. Figure 9 shows the levels of IL-6 in the serum of one group of patients before and after treatment. It can be seen that after treatment (post 2nd and post 5th) the levels of IL-6 are reduced in the patients' sera (n=4). Figure 10 shows the levels of IFN-γ in the serum of one group of patients before and after treatment. It can be seen that after treatment (post 2nd and post 5th) the levels of IFN-γ are reduced in the patients' sera.

### Example 5: The stabilised complex of the invention leads to induction of CRH

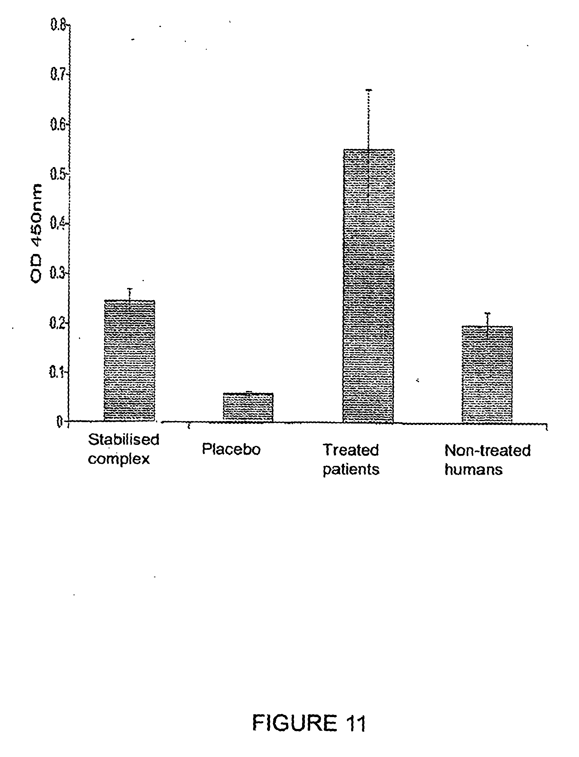

Figure 11 shows firstly, the increased presence of CRH in the stabilised complex of the invention by comparison with a placebo (human albumin at a concentration of 4.5 mg/ml); and secondly, the increase in CRH expression in patients treated with the stabilised complex of the invention by comparison with the non- treated individuals. This latter is evidence for the induction of CRH in patients in response to treatment with the stabilised complex of the invention.

All determinations were made in triplicate +/- standard deviations, These data are representative of at least 3 separate experiments. Control individuals n=4; treated patients n= 13. The data indicate that the stabilised complex of the invention induces the expression and release of CRH and hence POMC peptides in the patient, which then transform the patients' immunological profile from a TH-1 pro-inflammatory profile to a predominantly TH-2 anti-inflammatory profile.

### Example 6: The stabilised complex of the invention leads to an increase in CRH levels in patients with diffuse systemic sclerosis

A double-blind placebo control trial was carried out on patients with diffuse systemic sclerosis. The absolute levels of CRH (in micrograms per millilitre) were measured prior to treatment (baseline) and at 26 weeks following treatment. Treatment was carried out in a double-blind placebo control trial, with one group of subjects receiving the stabilised complex of the invention and the other (control) group receiving a placebo (human albumin at a concentration of 4.5 mg/ml). The patients received the treatment twice-weekly

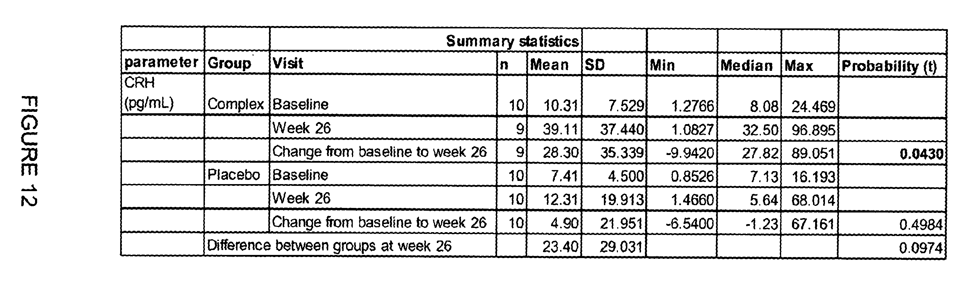

Figure 12 shows a three-fold increase (paired T-test, p<0.043) in CRH levels of the patients treated with the stabilised complex of the invention by comparison with those receiving the placebo.

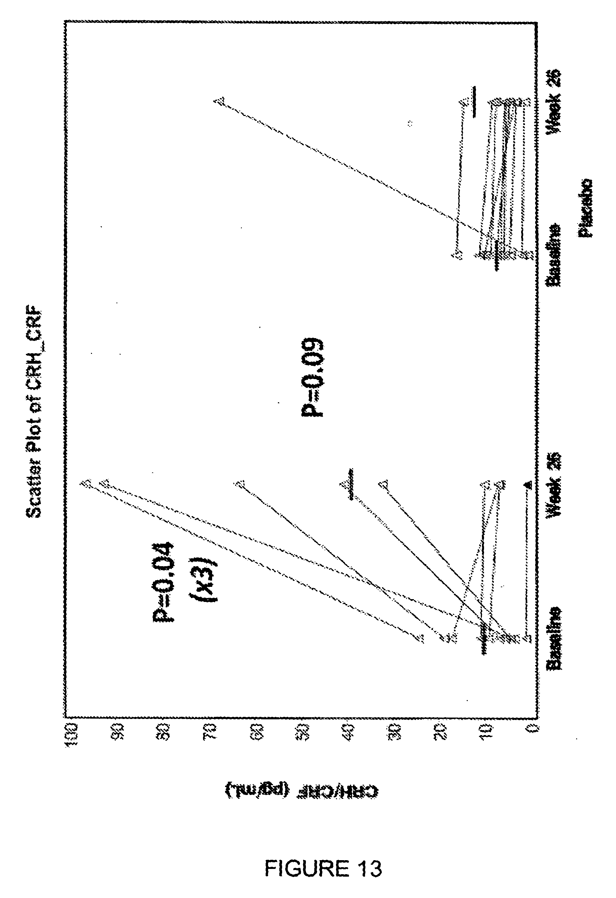

Figure 13 shows a plot of the results over the 26 weeks. The difference between the baseline (pre-treatment) level of CRH and the final level of CRH for each patient is shown graphically. The change in average level of CRH between the baseline and week 26 (shown by a bar) is significantly greater for the stabilised complex of the invention than for the placebo (P=0.04).

### Example 7: The stabilised complex of the invention leads to an increase in CRH levels in patients with progressive multiple sclerosis

A double-blind placebo control trial was carried out on patients with progressive multiple sclerosis. The absolute levels of CRH (in micrograms per millilitre) were measured prior to treatment (baseline) and at 4 weeks following treatment. Treatment was carried out in a double-blind placebo cross-over trial, with each group of patients receiving a twice-weekly treatment of either the stabilised complex of the invention or a placebo (human albumin at a concentration of 4.5 mg/ml) for four weeks, followed by a 6 week washout period, following which those patients previously receiving the placebo were administered the stabilised complex of the invention twice-weekly for 4 weeks; and those patients previously receiving the stabilised complex of the invention were administered the placebo twice-weekly for 4 weeks.

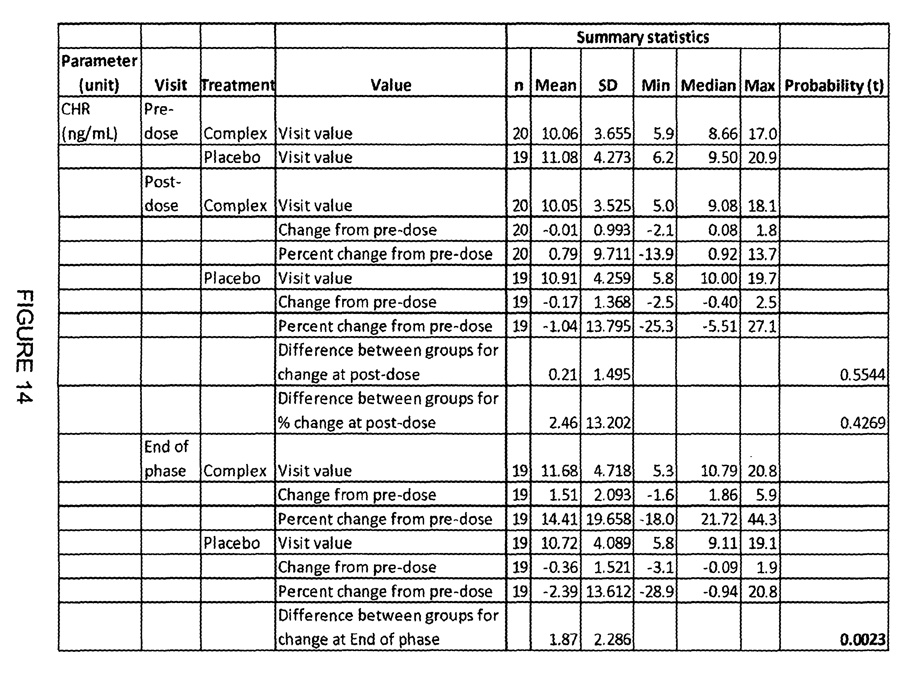

Figure 14 shows that for both groups, there was a significant difference (paired T-test, p<0.0023) in levels of CRH following administration of the stabilised complex of the invention, by comparison with the level of CRH following administration of the placebo.

### Example 8: The stabilised complex of the invention can be used in treating Hepatitis C

The subject was a 20 year old female patient with a lifelong history of infection with the Hepatitis C virus (HCV), sub-group type 2b.

The subject had previously been treated with interferon (24 month-long regime, unsuccessful) and with pegylated interferon alpha-2a and ribavirin (18-month long regime, unsuccessful). Prior to this study, the subject had received no intervention for the preceding 3 years.

Tests confirmed the presence of hepatitis C using HCV antibody enzyme immunoassay and quantitative PCR (1.5*10⁶ IU/ml). No jaundice was observed (bilirubin levels of 10 µmol/litre) and transaminits was detected at a mildly elevated level (aparatate transaminase AST 33 IU/litre, alanine transaminase 58 IU/litre). Further questioning of the subject revealed clinical signs and symptoms including lethargy, weight loss, malaise, insomnia and fatigue.

A 1 ml aliquot of the stabilised complex of the invention (4.5 mg/ml concentration) was prepared as described above. This was delivered by intramuscular route on an every other day treatment regimen for an initial six month course.

At the end of this six months, HCV quantitative PCR showed a drop to 150,000 IU/ml. The dosing regimen was then increased to a daily delivery of the stabilised complex of the invention for a further six month course.

Subsequent HCV titres were measured at 90,000 IU/ml, falling to 8,000 IU/ml and then (at the end of the further six months) a negative HCV titre, according to quantitative PCT. Normal liver function tests were also observed at the end of the further six month course.

## Claims

1. A method of manufacturing a stabilised complex of CRH and alpha-2 macroglobulin, the method comprising:
(a) providing hyperimmune serum from an ungulate that has been immunised with an immunodeficiency virus;
(b) subjecting said hyperimmune serum to a microfiltration step that removes molecules having a size greater than 0.2 microns, thereby providing a micro-filtered serum;
(c) subjecting said micro-filtered serum to a nanofiltration step that removes molecules having a size greater than 35 nanometres, thereby providing a nano-filtered serum;
(d) aliquoting said nano-filtered serum into a vial, wherein said nano-filtered serum is in a form for administration to a patient and comprises the stabilised complex of CRH and alpha-2 macroglobulin;
wherein the serum remains unfrozen throughout steps (b) to (d).

2. The method of claim 1, wherein the serum is frozen after step (d) and thawed prior to patient administration, though with the proviso that said serum is not subjected to a subsequent freezing step prior to said patient administration.

3. The method of claim 1 or claim 2, wherein the nanofiltration step is carried out using a 35 nanometre filter, for example using a 35 nanometre hollow fibre filter.

4. The method of any one of claims 1 to 3, further comprising the step of adjusting the final protein concentration to 4-5 milligrams protein per millilitre; optionally wherein the hyperimmune serum is only frozen after said adjusting of the final protein concentration.

5. The method of any one of claims 1 to 4, wherein exposure to ambient temperature is strictly minimised at all stages of the method.

6. A formulation comprising a stabilised complex of CRH and alpha-2 macroglobulin, wherein the formulation is produced by the method of any one of claims 1 to 5; optionally wherein the formulation comprises more than 50,000 pg/ml of alpha-2 macroglobulin.

7. A formulation comprising a stabilised complex of corticotropin releasing hormone (CRH) and alpha-2 macroglobulin, wherein the formulation comprises more than 50,000 pg/ml of alpha-2 macroglobulin.

8. The formulation of claim 6 or claim 7, comprising between 100,000 pg/ml and 150,000 pg/ml of alpha-2 macroglobulin; optionally wherein the formulation further comprises 80-120 pg/ml of CRH; optionally wherein the formulation comprises 90-110 pg/ml of CRH.

9. The formulation of any one of claims 6 to 8, wherein the CRH is non-human.

10. The formulation of any one of claims 6 to 9, further comprising one or more stabilisers; optionally wherein the one or more stabiliser is selected from fibronectin and albumin.

11. The formulation of any one of claims 6 to 10, further comprising a pro-opiomelanocortin (POMC) peptide; optionally at a concentration of at least 140pmol/L.

12. The formulation of claim 11, wherein the POMC peptide is non-human.

13. The formulation of any one of claims 6 to 12, further comprising one or more of vasopressin, ACTH, MSH such as alpha-MSH,beta-MSH, and gamma-MSH, LPH such as beta-LPH and gamma-LPH, beta-endorphin, enkephalin such as met-enkephalin and leu-enkephalin, CLIP, and Lipotrophin-gamma; and optionally further comprising CRH binding protein (CRH-BP).

14. The formulation of claim 13, comprising less than 50 pg/ml of CRH-BP.

15. The formulation of any one of one of claims 6 to 14, for use in prevention or treatment of one or more diseases selected from Alzheimer's disease; systemic sclerosis (SSc); multiple sclerosis; rheumatoid arthritis; optic neuritis; motor neuron disease; hepatitis, in particular hepatitis C; autoimmune diseases including lupus, psoriasis, eczema, thyroiditis, and polymyositis; axonal or nerve damage; cancers, in particular myelomas, melanomas, and lymphomas; neural disorders, both demyelinating and non-demyelinating; Parkinson's disease; inflammatory conditions; obesity; nerve conduction disorders; and sexual dysfunction, in particular erectile dysfunction.

## Patentansprüche

1. Verfahren zur Herstellung eines stabilisierten Komplexes von CRH und Alpha-2-Makroglobulin, wobei das Verfahren Folgendes beinhaltet:
(a) Bereitstellen eines Hyperimmunserums von einem Huftier, das mit einem Immundefizienzvirus immunisiert wurde;
(b) Unterziehen des genannten Hyperimmunserums einem Mikrofiltrationsschritt, bei dem Moleküle mit einer Größe von mehr als 0,2 Mikron entfernt werden, so dass ein mikrofiltriertes Serum erhalten wird;
(c) Unterziehen des genannten mikrofiltrierten Serums einem Nanofiltrationsschritt, bei dem Moleküle mit einer Größe von mehr als 35 Nanometern entfernt werden, so dass ein nanofiltriertes Serum erhalten wird;
(d) Aliquotieren des genannten nanofiltrierten Serums in eine Phiole, wobei das genannte nanofiltrierte Serum in einer Form zum Verabreichen an einen Patienten vorliegt und den stabilisierten Komplex von CRH und Alpha-2-Makroglobulin beinhaltet;
wobei das Serum während der Schritte (b) bis (d) ungefroren bleibt.

2. Verfahren nach Anspruch 1, wobei das Serum nach Schritt (d) eingefroren und vor dem Verabreichen an den Patienten aufgetaut wird, jedoch mit der Maßgabe, dass das genannte Serum vor der genannten Verabreichung an den Patienten keinem anschließenden Gefrierschritt unterzogen wird.

3. Verfahren nach Anspruch 1 oder Anspruch 2, wobei der Nanofiltrationsschritt mit einem 35-Nanometer-Filter durchgeführt wird, z.B. mit einem 35-Nanometer-Hohlfaserfilter.

4. Verfahren nach einem der Ansprüche 1 bis 3, das ferner den Schritt des Einstellens der endgültigen Proteinkonzentration auf 4-5 Milligramm Protein pro Milliliter beinhaltet; wobei das Hyperimmunserum optional erst nach dem genannten Einstellen der endgültigen Proteinkonzentration eingefroren wird.

5. Verfahren nach einem der Ansprüche 1 bis 4, wobei ein Kontakt mit Umgebungstemperatur in allen Stadien des Verfahrens strengstens minimiert wird.

6. Formulierung, die einen stabilisierten Komplex von CRH und Alpha-2-Makroglobulin beinhaltet, wobei die Formulierung mit dem Verfahren nach einem der Ansprüche 1 bis 5 produziert wird; wobei die Formulierung optional mehr als 50.000 pg/ml Alpha-2-Makroglobulin beinhaltet.

7. Formulierung, die einen stabilisierten Komplex von Corticotropin freisetzendem Hormon (CRH) und Alpha-2-Makroglobulin beinhaltet, wobei die Formulierung mehr als 50.000 pg/ml Alpha-2-Makroglobulin beinhaltet.

8. Formulierung nach Anspruch 6 oder Anspruch 7, die zwischen 100.000 pg/ml und 150.000 pg/ml Alpha-2-Makroglobulin beinhaltet; wobei die Formulierung optional ferner 80-120 pg/ml CRH beinhaltet; wobei die Formulierung optional 90-110 pg/ml CRH beinhaltet.

9. Formulierung nach einem der Ansprüche 6 bis 8, wobei das CRH nicht menschlich ist.

10. Formulierung nach einem der Ansprüche 6 bis 9, die ferner einen oder mehrere Stabilisatoren beinhaltet; wobei die ein oder mehreren Stabilisatoren optional aus Fibronektin und Albumin ausgewählt sind.

11. Formulierung nach einem der Ansprüche 6 bis 10, die ferner ein Proopiomelanocortin-(POMC)-Peptid beinhaltet; optional mit einer Konzentration von wenigstens 140 pmol/l.

12. Formulierung nach Anspruch 11, wobei das POMC-Peptid nicht menschlich ist.

13. Formulierung nach einem der Ansprüche 6 bis 12, die ferner eines oder mehrere aus Vasopressin, ACTH, MSH wie Alpha-MSH, Beta-MSH und Gamma-MSH, LPH wie Beta-LPH und Gamma-LPH, Beta-Endorphin, Enkephalin wie Met-Enkephalin und Leu-Enkephalin, CLIP und Lipotrophin-Gamma beinhaltet; und optional ferner CRH-Bindungsprotein (CRH-BP) beinhaltet.

14. Formulierung nach Anspruch 13, die weniger als 50 pg/ml CRH-BP beinhaltet.

15. Formulierung nach einem der Ansprüche 6 bis 14 zur Verwendung bei der Verhütung oder Behandlung von einer oder mehreren Krankheiten, die ausgewählt sind aus: Alzheimer-Krankheit; systemischer Sklerose (SSc); multipler Sklerose; Rheumatoid-Arthritis; optischer Neuritis; Motorneuron-Krankheit; Hepatitis, insbesondere Hepatitis C; Autoimmunkrankheiten wie Lupus, Psoriasis, Ekzem, Thyroiditis und Polymyositis; Axon- oder Nervenschaden; Krebs, insbesondere Myelome, Melanome und Lymphome; Nervenstörungen, sowohl demyelinisierend als auch nicht demyelinisierend; Parkinson-Krankheit; Entzündungszuständen; Fettleibigkeit; Nervenleitungsstörungen; und sexuelle Dysfunktion, insbesondere Erektionsstörungen.

## Revendications

1. Procédé de fabrication d'un complexe stabilisé de CRH et d'alpha-2 macroglobuline, le procédé comprenant :
(a) la fourniture de sérum hyperimmun provenant d'un ongulé qui a été immunisé avec un virus d'immunodéficience ;
(b) le fait de soumettre ledit sérum hyperimmun à une étape de microfiltration qui élimine les molécules ayant une taille supérieure à 0,2 micron, en produisant ainsi un sérum microfiltré ;
(c) le fait de soumettre ledit sérum microfiltré à une étape de nanofiltration qui élimine les molécules ayant une taille supérieure à 35 nanomètres, en produisant ainsi un sérum nanofiltré ;
(d) le fait de verser une aliquote dudit sérum nanofiltré dans un flacon, ledit sérum nanofiltré étant sous une forme destinée à une administration à un patient et comprenant le complexe stabilisé de CRH et d'alpha-2 macroglobuline ;
le sérum restant non congelé à travers les étapes (b) à (d).

2. Procédé selon la revendication 1, dans lequel le sérum est congelé après l'étape (d) et décongelé avant l'administration au patient, sous réserve toutefois que ledit sérum ne soit pas soumis à une étape de congélation ultérieure avant ladite administration au patient.

3. Procédé selon la revendication 1 ou la revendication 2, dans lequel l'étape de nanofiltration est réalisée en utilisant un filtre de 35 nanomètres, par exemple en utilisant un filtre à fibres creuses de 35 nanomètres.

4. Procédé selon l'une quelconque des revendications 1 à 3, comprenant en outre l'étape d'ajustement de la concentration finale en protéines jusqu'à 4-5 milligrammes de protéines par millilitre ; optionnellement dans lequel le sérum hyperimmun est congelé seulement après ledit ajustement de la concentration finale en protéines.

5. Procédé selon l'une quelconque des revendications 1 à 4, dans lequel l'exposition à la température ambiante est strictement minimisée à tous les stades du procédé.

6. Formulation comprenant un complexe stabilisé de CRH et d'alpha-2 macroglobuline, la formulation étant produite par le procédé selon l'une quelconque des revendications 1 à 5 ; la formulation comprenant optionnellement plus de 50 000 pg/ml d'alpha-2 macroglobuline.

7. Formulation comprenant un complexe stabilisé d'hormone de libération de la corticotrophine (CRH) et d'alpha-2 macroglobuline, la formulation comprenant plus de 50 000 pg/ml d'alpha-2 macroglobuline.

8. Formulation selon la revendication 6 ou la revendication 7, comprenant entre 100 000 pg/ml et 150 000 pg/ml d'alpha-2 macroglobuline ; la formulation comprenant optionnellement en outre 80-120 pg/ml de CRH ; la formulation comprenant optionnellement 90-110 pg/ml de CRH.

9. Formulation selon l'une quelconque des revendications 6 à 8, dans laquelle la CRH est non humaine.

10. Formulation selon l'une quelconque des revendications 6 à 9, comprenant en outre un ou plusieurs stabilisants ; lesdits un ou plusieurs stabilisants étant optionnellement sélectionnés parmi la fibronectine et l'albumine.

11. Formulation selon l'une quelconque des revendications 6 à 10, comprenant en outre un peptide de proopiomélanocortine (POMC), optionnellement à une concentration d'au moins 140 pmol/l.

12. Formulation selon la revendication 11, dans laquelle le peptide de POMC est non humain.

13. Formulation selon l'une quelconque des revendications 6 à 12, comprenant en outre une ou plusieurs de la vasopressine, de l'ACTH, d'une MSH telle que l'alpha-MSH, la bêta-MSH, et la gamma-MSH, d'une LPH telle que la bêta-LPH et la gamma-LPH, de la bêta-endorphine, d'une enképhaline telle que la mét-enképhaline et la leu-enképhaline, du corticotropin-like peptide (CLIP), et de la lipotrophine gamma ; et comprenant optionnellement en outre la protéine de liaison de la CRH (CRH-BP).

14. Formulation selon la revendication 13, comprenant moins de 50 pg/ml de CRH-BP.

15. Formulation selon l'une quelconque des revendications 6 à 14, destinée à une utilisation dans la prévention ou le traitement d'une ou plusieurs pathologies sélectionnées parmi la maladie d'Alzheimer ; la sclérose systémique (SSc) ; la sclérose en plaques ; la polyarthrite rhumatoïde ; la névrite optique ; la maladie neuromotrice ; l'hépatite, en particulier l'hépatite C ; des maladies auto-immunes comprenant le lupus, le psoriasis, l'eczéma, la thyroïdite, et la polymyosite ; les lésions axonales ou nerveuses ; les cancers, en particulier les myélomes, les mélanomes, et les lymphomes ; les affections neurales, aussi bien avec démyélinisation que sans démyélinisation ; la maladie de Parkinson ; les affections inflammatoires ; l'obésité ; les troubles de la conduction nerveuse ; et les dysfonctionnements sexuels, en particulier le dysfonctionnement érectile.
